# EUROPEAN PATENT SPECIFICATION

(11) **EP 3 102 668 B1**
(45) Date of publication and mention of the grant of the patent: **25.04.2018**
(21) Application number: 15707814.8
(22) Date of filing: 03.02.2015
(51) Int. Cl.: C12M 3/08, G01N 33/49, G01N 33/50, A61B 10/00, A61B 5/145, A61B 5/15

(54) **AN APPARATUS FOR THE SAMPLING AND PREPARATION OF BIOMEDICAL SAMPLES IN A CLOSED STERILE ENVIRONMENT**
VORRICHTUNG ZUR PROBENAHME UND AUFBEREITUNG VON BIOMEDIZINISCHEN PROBEN IN EINER GESCHLOSSENEN STERILEN UMGEBUNG
APPAREIL POUR L'ÉCHANTILLONNAGE ET LA PRÉPARATION D'ÉCHANTILLONS BIOMÉDICAUX DANS UN ENVIRONNEMENT STÉRILE FERMÉ

(30) Priority: 06.02.2014 IT FI20140027
(43) Date of publication of application: 14.12.2016
(73) Proprietor: Azienda Ospedaliero-Universitaria Careggi, 50134 Firenze (IT)
(72) Inventor: MIRABELLA, Carlo, I-52044 Cortona (Arezzo) (IT)
(74) Representative: Brazzini, Silvia
(86) International application number: PCT/IB2015/050807
(87) International publication number: WO 2015/118440

(56) References cited:
- WO-A1-83/03057
- GB-A- 989 885
- US-A- 3 800 780
- US-A- 4 134 512
- US-A1- 2005 084 961
- US-A1- 2013 203 175

## Description

### Field of the invention

The present invention concerns the field of medical apparatuses, and more specifically its object is an apparatus for sampling and preparing biomedical samples in a closed sterile environment.

### State of the art

The collection of biological or chemical material in a test tube or in a bag is common practice in medicine, biology and clinical chemistry for storing and/or handling multiple biological and chemical substances.

The devices used, test tubes or bags, which must ensure the integrity of the content, are generally sterile and equipped with a plug, usually made from plastic material and applied by pressing or screwed, suitable for separating the content from external contaminating agents. The opening of the test tube or bag, for example to introduce a biological or chemical sample therein or to withdraw from it, jeopardises the sterility inside it and the integrity of the sample, unless in environments considered to have extremely low chemical, bacterial and particle contamination (Work class ISO6 - Pharmaceutical Class A).

On the other hand, in environments with uncontrolled contamination and therefore also in normal chemical and/or biological laboratories and in sanitary environments, the practice of filling the closed and sterile test tubes or bags normally foresees that the chemical or biological materials be introduced into the device through a hole made with a needle through the plug made from plastic material of the test tube or bag. Through the needle, the material, for example contained in a syringe or in a bag or in another container connected to the needle itself, can be injected inside the device without opening the plug. The practice of perforating the plug with a needle connected to the external container is generally adopted also to recover the material from inside the test tube or bag without opening the plug, to limit as much as possible the contamination of the chemical or biological sample by the external environment.

During the step of introducing and withdrawing the sample, further precautions are generally adopted to limit the risks of contamination; in particular, to perforate the plug needles coated in plastic material are used and, both the surface of the needle and that of the plug intended to be perforated, are sanitised beforehand with disinfectant substances.

On the market there are also test tubes, known by the trade name Vacutainer® and used for example in drawing blood, with which it is possible to introduce a sample inside the test tube without perforating the plug; the vacuum inside these test tubes is indeed sufficient to draw the sample inside the test tube. In order to take the sample, on the other hand, the plug must be opened or perforated again. Such test tubes therefore ensure sterility only in the step of introducing the sample and during storage, whereas sterility is completely lost at the moment when the sample must be taken.

The US patent No. US 4,134,512 describes a stopper for removable mounting in an evacuated tube for collecting fluid samples.

The UK patent No. GB 989,885 describes an apparatus for obtaining separate donation volumes of blood in a sterile manner, and the related method.

None of the currently known systems, despite the various precautions to minimise the risks of contamination, can however guarantee a route of use that is completely sterile: such systems, indeed, are unable to rule out a *priori* a possible environmental contamination, being largely dependent on the expertise of the operator and on the environmental conditions to which the test tube or bag are exposed. For example, in the very common practice of centrifuging samples in a test tube, the container is subjected to whirling flows of uncontrolled air, particularly exposed to environmental contaminations.

Therefore there is still the problem of having an effective system for ensuring the integrity and sterility of biomedical samples in laboratory containers, during all of the handling steps of the samples and containers.

### Summary of the invention

The purpose of the present invention is therefore to provide an apparatus for handling sterile test tubes bags, or other containers, which allow carrying out all of the operations of withdrawing the sample, introducing into the container, if necessary preparing the container with the sample for subsequent processing, without the sample itself coming into contact with the external environment thanks to a perfectly closed and sterile system.

A further purpose of the invention is to provide an apparatus that allows carrying out the same operations mentioned above, with many test tubes or bags or other containers connected together.

These and other purposes are accomplished by the apparatus according to the present invention for sampling and preparing samples in test tubes, bags and other containers, which radically eliminates the risk of contamination of the biological material contained in them, both in the filling step and in the subsequent steps of processing and withdrawal. The essential characteristics of the present apparatus, which constitutes a true sterile chamber, are defined in the independent claims attached hereto.

Further important characteristics are included in the dependent claims.

The characteristics and advantages of the apparatus according to the present invention will become clearer from the following description of embodiments thereof, given as an example and not for limiting purposes, with reference to the attached figures, in which:
- figure 1 is a schematic representation of a first embodiment of the apparatus of the invention;
- figure 2 is a view of the present apparatus in the embodiment of figure 1;
- figure 3 is a schematic representation of a second embodiment of the apparatus of the invention;
- figure 4 is a view of the present apparatus in the embodiment of figure 2.

With reference to the aforementioned figures, the apparatus according to the invention comprises: one or more sampling means 1 of biological samples, for example of blood fluids from patients, one or more collecting means 2 of the aforementioned samples for diagnostic use, one or more cylindrical containers 3 for processing the aforementioned samples, housed inside cylindrical bodies 4, which are hollow and have a greater diameter than the containers 3, at the upper end of which a hollow element 5 is fixed, facing the inside of the container and such as to place the container itself in fluid communication with the outside, and one or more collecting means 6 for diagnostic or therapeutic use of the processed aforementioned samples, coming from the containers 3, such means being connected together by a rigid or flexible device able to distribute fluids, for example a system of pipes that allows the flow of the samples from the sampling means 1 to the collecting means 2, or from the sampling means 1 to the containers 3 and, from these, to the collecting means 6, creating an environment that is closed and able to maintain sterile internal conditions.

Such a device in particular forms a distribution network of the samples taken with the sampling means 1, towards the different collecting means 2 and 6; in such a distribution network it is possible to distinguish a first branch PR that takes the sample taken by the sampling means 1 to the collecting means 2, where a first portion of the sample is collected to be optionally eliminated or conserved separately for diagnostic use, and a second branch SR that takes the sample taken by the sampling means 1 to the containers 3 for the processing of the samples themselves and then from these to the collecting means 6. The distribution network of the fluid sample can comprise fittings able to let the sample flow in the desired direction distribute it in the containers; for example, a W-fitting will be present in the second branch SR for the distribution of the sample in the container 3, or in the containers 3 if more than one, and for their return from these to the collecting means 6.

The flow of the sample can also be regulated by a series of devices, like for example nonreturn valves that allow the sample to flow inside them only in one direction, or break valves or similar devices that allow the sample to flow inside them only if triggered by a mechanical action, which can be manual or automatic. The present apparatus can for example comprise a nonreturn valve 7 in the second branch SR at the entry into the collecting means 6, to prevent the return of the sample collected in them back towards the distribution network. The present apparatus can, on the other hand, comprise a break valve 8 to control the drawing of the sample flow in the desired direction, advantageously, in the first branch PR between sampling means 1 and collecting means 2, in the second branch SR to draw the sample flow towards the containers 3 and, from these, towards the collecting means 6. There can also be one or more flow interruption means 9, manual or automatic, one-click or multi-click, reopenable or not openable, in the present apparatus, for example clamps, able for example to interrupt the flow of sample in the first branch PR towards the collecting means 2 during the flow of sample to be processed towards the containers 3. Between the flow interruption means, in the present apparatus it is also possible to use antibacterial valves, valves that close automatically upon extraction of the infusion tubing in which the sample slides; tubes made from thermoweldable plastic material can also be used, so as to be able to interrupt the flow in a certain position using a heat sealing tool that interrupts the flow cutting and sealing the tube at the end.

The present apparatus can comprise a single container 3 or more than one container 3, in the case wherein the amount of biological material to be processed and collected is greater; in other words, the number of containers 3 is proportional to the amount of material that it is wished to process and collect.

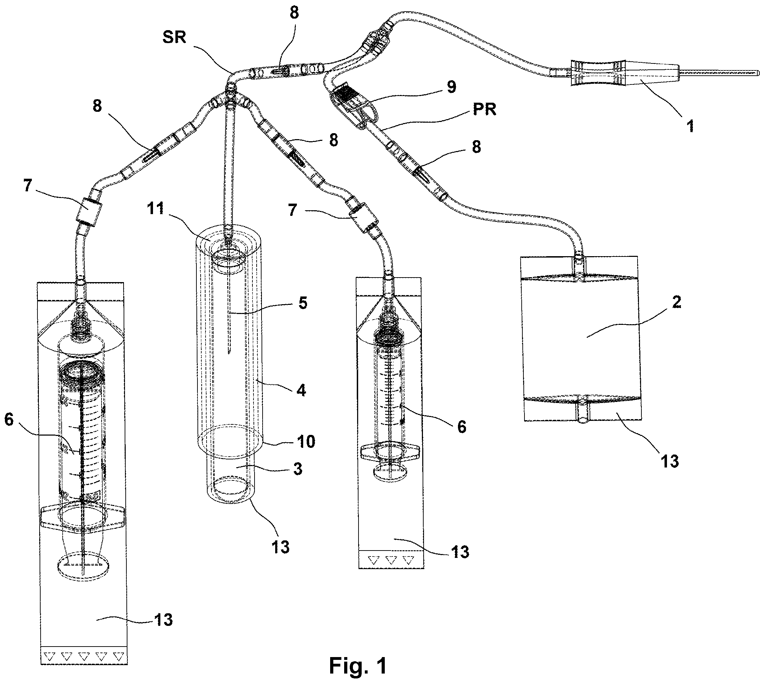

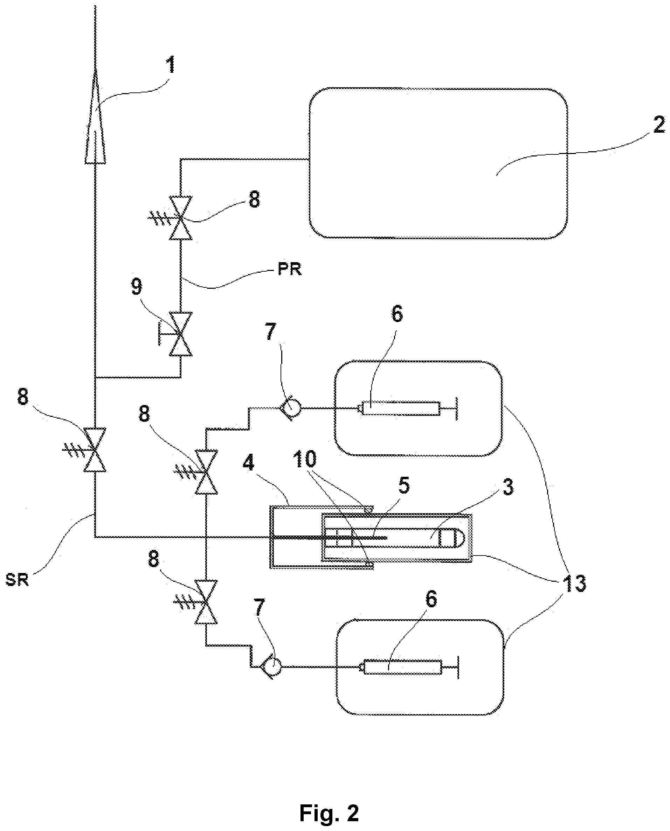

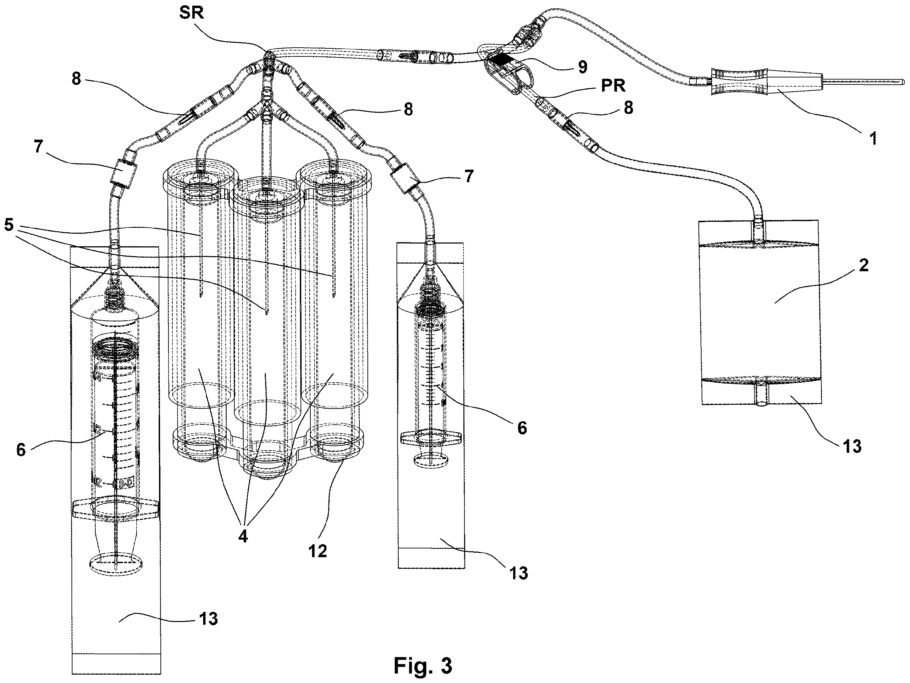

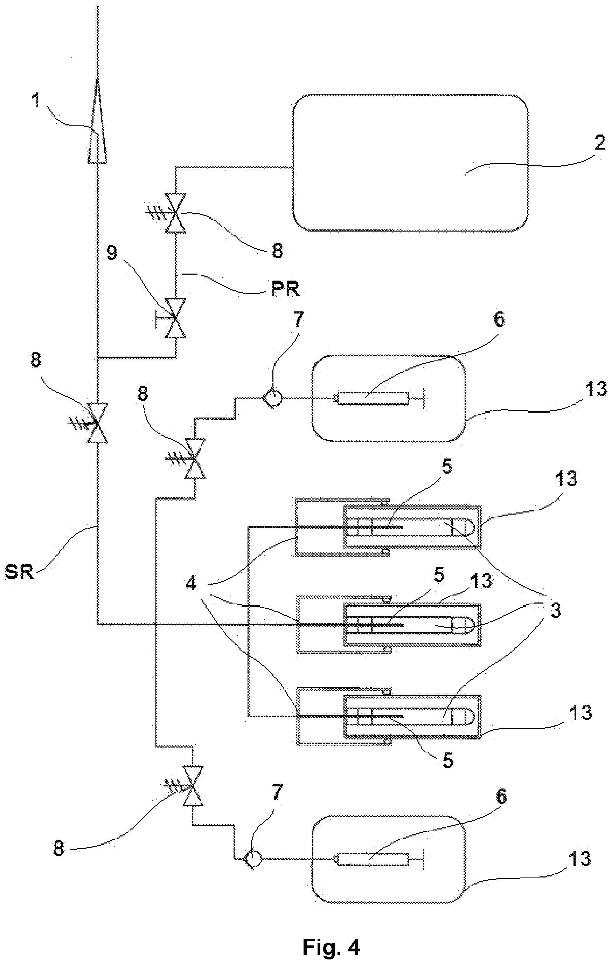

Figures 1 and 2 illustrate a first embodiment of the present apparatus, in which there is a single container 3, whereas figures 3 and 4 illustrate a second embodiment of the apparatus of the invention, in which there are three containers 3.

Preferably, all of the components of the present apparatus, and in particular the containers 3 and the collecting means 2 and 6 are each enclosed inside a casing sterile 13, which keeps them individually in a protected and sterile atmosphere. In this way, the possible separation of a component from the apparatus, for example of a collecting means, does not run risks of contaminations from the outside nor losses of material that could place the operator at risk.

By sampling means 1 according to the invention we mean any device able to take biological samples, in particular a butterfly needle with infusion tubing like those typically used for taking blood samples from patients. Collecting means 2 of the present apparatus, on the other hand, can be collecting bags, optionally equipped with a sterile glass or plastic tube with hermetic closure inside which the vacuum can be created to facilitate the drawing of a fluid through it, according to the system commonly used for sampling blood and known by the trade name Vacutainer®. The collecting means 6 can for example be syringes, preferably packaged in sterile casings.

According to the invention, the containers 3 for processing the fluid samples taken can be made from glass or plastic, rigid or flexible, substantially cylindrical in shape, closed on the bottom, rounded or conical according to requirements, and suitably sealed in the upper part, for example by a plug and/or by gaskets, which ensure the hermetic seal of the container. Inside them, the containers 3 can receive substances to promote the subsequent processing for which the sample is intended, for example substances for promoting the stratification of the biological samples for subsequent centrifuging. The hollow bodies 4, also cylindrical in shape and having a greater diameter than the container 3, are able to receive at least part of the container 3 with the relative casing 13 inside them; thanks to gaskets 10 arranged between the inner surface of the hollow bodies 4 and the outer surface of the containers 3, the hermetic seal between the surfaces and the insulation of the inside of the hollow body from the external environment is ensured. The hollow elements 5 arranged on the top of the bodies 4 are for example hollow needles made from metal or plastic, through which the sample taken flows inside the container 3, and by means of which the sample collected in the container 3, after possible stratification or other treatments, can be drawn and transferred into the collecting means 6. Such drawing from the container 3 by means of the element 5 can be carried out at various heights, for example so as to draw sample portions from different layers formed following stratification of the original sample; this is obtained thanks to the sliding upwards or downwards of the hollow body 4 on the container 3 coated by the sterile casing 13, made possible by the upper closure 11 of container and hollow body, for example plug and gasket, which has a cap structure, telescopic or in any case flexible and elongatable whilst still maintaining the hermetic closure of container and hollow body. The cap 11 that closes the hollow body 4 on top is centrally perforated by the hollow element 5 facing the inside of the container and able to put it into fluid communication with the outside of the container, and it can stretch upwards or downwards to vary the height of the hollow element 5, integral with it, inside the container 3.

The apparatus of the invention can be used to draw biological fluid samples, from patients as well as from containers where the sample may be stored. The drawing of the fluid sample through the sampling means 1, and its entry and circulation in the distribution network of the present apparatus, is triggered thanks to the inlet pressure, be it due to the body pressure of the patient or to the geodetic height of the container from which the sample is taken, or furthermore by a thrusting tank arranged upstream of the collecting means 2 and/or of the container 3 so as to promote its complete filling. The thrusting tank is preferably equipped with a nonreturn valve. For the purposes of microbiological safety, the first part of the sample taken, which may be contaminated, is always sent towards the collecting means 2, consisting of one or more "satellite bags", and then only used for diagnostic purposes; in order to do this, it is sufficient to act on the flow control valves present in the apparatus, for example opening only one break valve present in the first branch PR and keeping a break valve present in the second branch SR closed. Once the first portion of the sample is collected, the first branch PR is closed to the entry of other material, for example by means of a clamp or other flow interrupter, and, on the other hand, the second branch SR is opened triggering the entry of the sample that is made to flow towards the container 3, or containers 3 if there is more than one container, directly or through one or more thrusting tanks if present. Once the desired amount of sample has been collected in such containers, the flow is interrupted, and the containers are subjected to the processing required on each occasion, individually after having extracted them from the apparatus, still enclosed in their sterile casing 13, or even connected to the other components of the present apparatus. In the case in which for example it is wished to subject the samples collected to stratification, or the containers are left to rest in vertical position obtaining stratification by sedimentation, or the apparatus as a whole can be arranged in a suitable basket of a centrifuge; possible chemical or heat treatments can aid the mechanical action, promoting the stratification of the materials of different specific weight contained in the sample, for example an upper layer of serum and a lower layer of corpuscular part for blood samples.

In order to allow axial separation of the components during centrifuging, it is necessary to avoid inverting the containers 3; a support made from rigid plastic material or from metal able to support the containers 3, keeping them joined together and creating a base that prevents it from being inverted, is indicated with 12 in figure 3.

The sample thus treated, or just one of the layers that has formed following stratification, can at this point be taken from the container 3 by means of the hollow element 5 and sent to the collecting means 6. Once the collection is complete, such means can be removed from the apparatus still maintaining the sterility thanks to the sterile casing 13 in which they are packaged. A nonreturn valve arranged upstream of the collecting means and the heat sealing of the tube between nonreturn valve and collecting means, prevent the reflux of the sample towards the containers 3 and/or the loss of sample from the apparatus also for the sake of the safety of the operator. Advantageously, in the case in which the sample collected must be used for therapeutic purposes, the collecting means 6 can be a syringe, able to be directly used, once separated from the apparatus, for the parenteral administration of the sample to a patient that needs it; moreover, in this case, one or more therapeutic substances can be added beforehand in the collecting means 6, like for example components of the extracellular matrix such as hyaluronic acid.

The operations of controlling, drawing or interrupting the flow inside the present apparatus through actuation of suitable valves, as well as drawing the processed sample from the container 3 to send to the relative collecting means 6, and the same processing procedures of the sample inside the container 3, can be completely manual, partially or totally automated and controlled by suitable software in an external control unit to the present apparatus.

According to a preferred embodiment of the present invention, the container 3, the hollow body 4, the sterile casings 13, the thrusting tanks if present, and/or the collecting means 2, 6 are made from transparent plastic material, so that the operator can constantly visually monitor the sample during the various operations inside the present apparatus. Preferably, on the external components, be they collecting means or sterile casings, it is possible to apply tags with bar codes or other identification means of the samples and of the type of component, for example if it is a collecting means 2 for diagnostic use or a collecting means 6 for clinical or therapeutic use, or it is possible to apply the signature of the patient where required, for example in the case of autologous clinical use of the fluids collected.

The characteristic of the present apparatus of being a closed system leads to the elimination or at least the reduction of operative steps that would expose the sample to the risk of environmental, chemical, organic and microbiological contamination; at the same time, safety is also increased for the operator against the risks linked to contamination by potentially infectious biological material.

With respect to the prior art the present apparatus is ready for use, since it is preassembled and contained in a casing 13 suitable for maintaining a sterile internal atmosphere. As a further precaution, in order to increase the safety of the operators and of the patient, the components to be used for sampling, collecting, stratification, fractioning and conservation of the biological samples, can in turn also be packaged inside sterile casings.

A further advantage of the present apparatus consists of the fact that, in the case in which it is necessary to process many biological samples collected, for example subjecting them to a stratification step, such samples are all grouped and joined together, and at the same time inserted in sterile packages; this allows them to be clearly identified by the operator and allows the reduction, if not the complete elimination, of errors. The reduction of errors by the operator is also ensured by the clear definition and separation of the phases thanks to the barrier system created by the present apparatus.

A further advantage of the present apparatus is the elimination, or at least the reduction of the accessories and/or tools that it is typically necessary to use to conserve sterility for example during transfer from one sterile environment to another, passing through a non-sterile environment. An example is the laminar flow cabinet, or microbiological safety cabinet, which protects against environmental contaminants thanks to contrasting flows of ultra-filtered air, which create a true protective barrier to anything that is positioned inside it; thanks to the present apparatus, complex and very expensive apparatuses like this are totally superfluous.

A further advantage of the apparatus of the invention is the reduction of the risk of losses of biological material from the samples, which are possible in particular during certain processing steps, in particular during centrifuging. Such losses could contaminate both the environment and possible other samples in adjacent containers. The present apparatus therefore ensures better protection from the risks of contamination of the sample both by external environmental agents and from other adjacent samples, and also protects the environment and the operator himself/herself from the risk of contamination by the samples being processed.

A further advantage of the present apparatus is that of being able to have spaces on the collecting means or the sterile casings for the application of tags carrying bar codes or other identification means of the samples collected or of the type of container on which they are applied, for example if it is a collecting means 2 for diagnostic use or a collecting means 6 for clinical or therapeutic use, or it is possible to apply the signature of the patient where required, for example in the case of autologous clinical use of the fluid samples collected.

In conclusion, summarising, the apparatus of the invention makes it possible to safely carry out the sampling and subsequent collection and processing for diagnostic and/or therapeutic use of biological samples, including body fluids, for example peripheral venous blood samples, medullary blood, and cerebrospinal fluid; biological samples in the present invention can also mean samples of biological material intended for autologous lipofilling. The present apparatus also has the following advantageous characteristics: all of the processing operations take place in a closed circuit, the sterile apparatus can in turn be packaged inside a sterile casing, the end product obtained from the processing carried out inside the present apparatus can also be easily conserved inside it and can be easily identified for safety and traceability.

The present invention has been described up to here with reference to its preferred embodiments. It should be understood that there can be other embodiments that derive from the same inventive core, all of which are encompassed by the scope of protection of the claims given hereafter.

## Claims

1. An apparatus for sampling, processing and collecting biomedical samples for a subsequent diagnostic, clinical and/or therapeutic use, in a closed sterile environment, said apparatus comprising: one or more sampling means (1) of biomedical samples, one or more collecting means (2) of said samples for diagnostic use, one or more containers (3) for processing of said samples, each housed inside a hollow body (4) having a diameter greater than the diameter of said containers (3) and able to sliding upwards and downwards on the outer surface of said containers, said hollow body (4) being closed at the top by a cap (11) centrally perforated by a hollow element (5) facing the inside of the container and able to put it into fluid communication with the outside, **characterized in that** said cap (11) is able to stretch upwards or downwards in order to vary the height of said hollow element (5) integral therewith, inside said container (3); and one or more collecting means (6) for clinical or therapeutic use of said processed samples coming from said containers (3), said means being connected between each other by a device for the distribution of fluids consisting of rigid or flexible pipes, which let said sample flow from said sampling means (1) to said collecting means (2) or from said sampling means (1) to said containers (3) and, from these, to said collecting means (6), thus creating an environment which is closed and able to maintain internal sterile conditions.

2. The apparatus according to claim 1, wherein said container (3) and/or said collecting means (2, 6) are individually enclosed in sterile casings (13).

3. The apparatus according to claim 1 or to claim 2, wherein said container (3), said hollow body (4), said sterile casings (13) and/or said collecting means (2, 6) are made by a transparent plastic material, and tags are affixed thereon with bar codes or other identification means of the sample contained therein and related information and/or the signature of the patient from which the sample has been taken.

4. The apparatus according to claim 1, wherein between the inner surface of said hollow body (4) and the outer surface of said container (3) gaskets (10) are placed, that guarantee the hermetic seal between said surfaces and the insulation of the interior of said hollow body from the external environment, while still leaving said hollow body free to slide on said container.

5. The apparatus according to any of the preceding claims, wherein said device for the distribution of fluids comprises flow control valves, capable of triggering the flow in a certain direction or to interrupt it.

6. The apparatus according to any of the preceding claims, wherein said biomedical samples are blood samples or other biomedical samples that have to be separated into liquid and corpuscular components by stratification, before clinical or therapeutic use, and wherein said hollow body (4), sliding on said container (3), does vary the height of said hollow element (5) inside said container depending on the position of the sample's layer that it is desired to take for collection in said collecting means (6).

7. The apparatus according to any of the preceding claims, further comprising an external control unit equipped with a software for the automatic management of all or part of the operations of flow control, retrieval or interruption inside the apparatus by actuating said valves, of the withdrawal of the processed sample from said container (3) for sending to said collecting means (6) and of the processing of said sample in the container (3).

## Patentansprüche

1. Vorrichtung zur Probennahme, Verarbeitung und Sammlung von biomedizinischen Proben in einer abgeschlossenen sterilen Umgebung für eine nachfolgende diagnostische, klinische und/oder therapeutische Verwendung, wobei die Vorrichtung enthält: eine oder mehrere Probenentnahmeeinrichtung(en) (1) für biomedizinische Proben, eine oder mehrere Sammlungseinrichtung(en) (2) für die Proben zur diagnostischen Verwendung, einen oder mehrere Behälter (3) zum Verarbeiten der Proben, wobei jeder innerhalb eines hohlen Körpers (4) untergebracht ist, der einen Durchmesser hat, der größer als der Durchmesser der Behälter (3) ist, und in der Lage ist, sich an der äußeren Oberfläche der Behälter aufwärts und abwärts zu verschieben, wobei der hohle Körper (4) an der Oberseite durch eine Kappe (11) verschlossen ist, die zentral durch ein hohles Element (5) perforiert ist, das der Innenseite des Behälters zugewandt ist und in der Lage ist, ihn in Fluidverbindung mit der Außenseite zu bringen, **dadurch gekennzeichnet, dass** die Kappe (11) in der Lage ist, sich aufwärts oder abwärts zu dehnen, um die Höhe des mit ihr integralen hohlen Elements (5) innerhalb des Behälters (3) zu variieren; und eine oder mehrere Sammlungseinrichtung(en) (6) klinische und/oder therapeutische Verwendung der verarbeiteten Proben von den Behältern (3) her kommen, wobei die Einrichtungen jeweils zwischen einander mit einer Einrichtung für die Verteilung von Fluiden bestehend aus steifen oder flexiblen Rohren verbunden sind, die die Probe von den Probenentnahmeeinrichtungen (1) zu den Sammlungseinrichtungen (2) oder von den Probenentnahmeeinrichtungen (1) zu den Behältern (3) und von diesen zu den Sammlungseinrichtungen (6) strömen lassen, womit eine Umgebung geschaffen ist, die geschlossen ist und in der Lage ist, die internen sterilen Zustände aufrecht zu erhalten.

2. Vorrichtung nach Anspruch 1, wobei der Behälter und/oder die Sammlungseinrichtungen (2, 6) einzeln in sterilen Hüllen (13) eingeschlossen sind.

3. Vorrichtung nach Anspruch 1 oder Anspruch 2, wobei der Behälter (3), der hohle Körper (4), die sterilen Hüllen (13) und/oder die Sammlungseinrichtungen (2, 6) aus einem transparenten Kunststoffmaterial hergestellt sind und Marken mit Barcodes oder anderen Identifikationseinrichtungen der darin enthaltenen Probe und zugehöriger Information und/oder der Signatur des Patienten daran befestigt sind, von dem die Probe genommen wurde.

4. Vorrichtung nach Anspruch 1, wobei zwischen der inneren Oberfläche des hohlen Körpers (4) und der äußeren Oberfläche des Behälters (3) Dichtungen (10) angeordnet sind, die die hermetische Abdichtung zwischen den Oberflächen und die Isolation des Inneren des hohlen Körpers von der äußeren Umgebung garantieren, während sie weiterhin den hohlen Körper sich frei auf dem Behälter verschieben lassen.

5. Vorrichtung nach einem der vorhergehenden Ansprüche, wobei die Vorrichtung zur Verteilung von Fluiden Strömungssteuerventile enthält, die zum Auslösen der Strömung in eine bestimmte Richtung oder zu ihrer Unterbrechung in der Lage sind.

6. Vorrichtung nach einem der vorhergehenden Ansprüche, wobei die biomedizinischen Proben Blutproben oder andere biomedizinische Proben sind, die vor einer klinischen oder therapeutischen Verwendung durch Schichtenbildung in flüssige und korpuskulare Komponenten getrennt werden müssen, und wobei der hohle Körper (4), der sich auf dem Behälter (3) verschiebt, die Höhe des hohlen Elementes (5) innerhalb des Behälters in Abhängigkeit von der Position der Schicht der Probe variiert, von der erwünscht ist, für die Sammlung in den Sammlungseinrichtungen (6) genommen zu werden.

7. Vorrichtung nach einem der vorhergehenden Ansprüche, wobei ferner eine externe Steuereinheit enthalten ist, die mit einer Software für die automatische Verwaltung aller oder eines Teils der Operationen der Strömungssteuerung, Wiedererlangung oder Unterbrechung innerhalb der Vorrichtung durch Betätigen der Ventile, von der Entnahme der verarbeiteten Probe von dem Behälter (3) zur Übermittlung zu den Sammlungseinrichtungen (6) und der Verarbeitung der Probe in dem Behälter (3) ausgestattet ist.

## Revendications

1. Un appareil pour l'échantillonnage, le traitement et la collecte d'échantillons biomédicaux pour un usage ultérieur diagnostique, clinique et/ou thérapeutique, dans un environnement stérile fermé, ledit appareil comprenant: un ou plusieurs moyens d'échantillonnage (1) d'échantillons biomédicaux, un ou plusieurs moyens collecteurs (2) desdits échantillons à usage diagnostique, un ou plusieurs récipients (3) pour traiter lesdits échantillons, chacun logé à l'intérieur d'un corps creux (4) de diamètre supérieur au diamètre desdits récipients (3) et apte à glisser vers le haut et vers le bas sur la surface extérieure desdits récipients, ledit corps creux (4) étant fermé au sommet par un capuchon (11) perforé centralement par un élément creux (5) tourné vers l'intérieur du récipient et apte à le mettre en communication fluidique avec l'extérieur, **caractérisé en ce que** ledit capuchon (11) est apte à s'étirer vers le haut ou vers le bas pour faire varier la hauteur dudit élément creux (5) solidairement avec celui-ci, à l'intérieur dudit récipient (3); et un ou plusieurs moyens de collecte (6) pour une utilisation clinique ou thérapeutique desdits échantillons traités provenant desdits récipients (3), lesdits moyens étant reliés entre eux par un dispositif de distribution de fluides constitué de tuyaux rigides ou flexibles, qui laissent ledit échantillon s'écouler desdits moyens d'échantillonnage (1) vers lesdits moyens de collecte (2) ou desdits moyens d'échantillonnage (1) vers lesdits récipients (3) et, à partir de ceux-ci, vers lesdits moyens de collecte (6), créant ainsi un environnement qui est fermé et capable de maintenir des conditions intérieures stériles.

2. L'appareil selon la revendication 1, dans lequel ledit récipient (3) et/ou lesdits moyens de collecte (2, 6) sont individuellement enfermés dans des enveloppes stériles (13).

3. L'appareil selon la revendication 1 ou la revendication 2, dans lequel ledit récipient (3), ledit corps creux (4), lesdites enveloppes stériles (13) et/ou lesdits moyens de collecte (2, 6) sont réalisés en un matériau plastique transparent, et des étiquettes sont apposées sur eux avec des codes barres ou autres moyens d'identification de l'échantillon qui y est contenu et des informations le concernant et/ou la signature du patient sur lequel l'échantillon a été prélevé.

4. L'appareil selon la revendication 1, dans lequel entre la surface intérieure dudit corps creux (4) et la surface extérieure dudit récipient (3) sont placés des joints (10) qui garantissent l'étanchéité entre lesdites surfaces et isolatent l'intérieur dudit corps creux de l'environnement extérieur, tout en laissant ledit corps creux libre de glisser sur ledit récipient.

5. L'appareil selon l'une quelconque des revendications précédentes, dans lequel ledit dispositif pour la distribution de fluides comprend des valves de régulation de débit, capables de déclencher l'écoulement dans une certaine direction ou de l'interrompre.

6. L'appareil selon l'une quelconque des revendications précédentes, dans lequel lesdits échantillons biomédicaux sont des échantillons sanguins ou d'autres échantillons biomédicaux qui doivent être séparés en liquide et composants corpusculaires par stratification, avant utilisation clinique ou thérapeutique, et dans lequel ledit corps creux (4), coulissant sur ledit récipient (3), fait varier la hauteur dudit élément creux (5) à l'intérieur dudit récipient en fonction de la position de la couche d'échantillon que l'on souhaite prendre pour la collecte dans lesdits moyens de collecte (6).

7. L'appareil selon l'une quelconque des revendications précédentes, comprenant en outre une unité de commande externe équipée d'un logiciel pour la gestion automatique de tout ou partie des opérations de contrôle, de prélèvement ou d'interruption de l'écoulement à l'intérieur de l'appareil par actionnement desdites valves, du retrait, hors du récipient (3), de l'échantillon traité pour l'envoyer auxdits moyens de collecte (6) et du traitement dudit échantillon dans le récipient (3).
